# EUROPEAN PATENT APPLICATION

(11) **EP 4 483 868 A1**
(43) Date of publication of application: **01.01.2025**
(21) Application number: 23756577.5
(22) Date of filing: 10.02.2023
(51) Int. Cl.: A61K 31/198, A61K 31/375, A61P 17/00, A61K 8/44, A61Q 19/08, A23L 33/175

(54) **COMPOSITION FOR PROMOTING COLLAGEN PRODUCTION COMPRISING AMIDATED AMINO ACID**

(30) Priority: 21.02.2022 KR 20220022559
(71) Applicant: Boo, Yong Chool, Daegu 42016 (KR)
(72) Inventor: BOO, Yong Chool, Daegu 41566 (KR); LEE, Ji Eun, Daegu 41566 (KR)
(74) Representative: JWP Patent & Trademark Attorneys
(86) International application number: PCT/KR2023/001982
(87) International publication number: WO 2023/158164

(57) **Abstract**

The present invention relates to a composition for promoting collagen production comprising amidated amino acids, and more specifically, to a composition that promotes collagen production in various cells such as fibroblasts, follicular cells, dermal papilla cells, endothelial cells, chondrocytes, muscle cells, and osteoblasts, thereby preventing, improving, or treating various diseases and lesions that may result from collagen reduction in the skin, hair, blood vessels, cartilage, tendons, connective tissues, muscles, bones, teeth, and periodontal tissues.

## Description

### TECHNICAL FIELD

This application claims priority to Korean Patent Application No. 10-2022-0022559, filed on February 21, 2022, the entirety of which is incorporated herein by reference.

The present invention relates to a composition for promoting collagen production comprising amidated amino acids, and more specifically, to a composition that promotes collagen production in various cells such as fibroblasts, follicular cells, dermal papilla cells, endothelial cells, chondrocytes, muscle cells, and osteoblasts, thereby preventing, improving, or treating various diseases and lesions that may result from collagen reduction in the skin, hair, blood vessels, cartilage, tendons, connective tissues, muscles, bones, teeth, and periodontium.

### BACKGROUND OF THE INVENTION

Collagen is the most abundant protein in animals, comprising 30% of the dry mass of skin. Collagen exists in the form of a triple helix, with its primary amino acid sequence repeating as glycine-proline-X or glycine-X-hydroxyproline, where X can be any amino acid other than glycine, proline, or hydroxyproline. To date, a total of 28 different types of collagen have been identified, with type I collagen being the most common, making up over 90% of human collagen. Normal human skin comprises various types of collagen, specifically 80-90% type I collagen, 8-12% type III collagen, and 5% type V collagen. Collagen, the main component of the extracellular matrix (ECM) in the dermis, provides structural support and maintains the firmness and elasticity of the skin.

Insufficient or excessive levels of dermal collagen are associated with various skin diseases and cosmetic issues. Collagen disorders can occur when there is a defect in collagen production and maturation due to genetic causes, when collagen production is inadequate due to a deficiency of ascorbic acid (AA), or when collagen production is excessively increased due to an autoimmune response.

Transforming Growth Factor-beta1 (TGF-β1) increases collagen production in cells and promotes cell proliferation and differentiation. Ascorbic acid (vitamin C), as an essential cofactor for prolyl hydroxylase and lysyl hydroxylase, plays a crucial role in the post-translational stage of collagen. Ascorbic acid also stimulates collagen gene transcription in cells.

Extracellular matrix proteins comprising collagen have a biased amino acid composition, thus the production of collagen by cells is influenced by the availability of specific amino acids. According to existing research reports, the external supply of proline increases collagen production in fibroblasts, and it is known that glycine has a greater impact on collagen production in joint cartilage cells compared to proline, lysine, and other amino acids.

However, the existing methods comprising TGF-β1, ascorbic acid, and free amino acids are insufficient in promoting collagen production, necessitating the development of novel substances that can more efficiently stimulate intracellular collagen synthesis.

### SUMMARY OF INVENTION

### PROBLEM TO BE SOLVED

Thus, the inventors of the present invention find a substance that can safely and very efficiently promote collagen production in various cells, discovered that amidated amino acids can exhibit such effects.

Therefore, the objective of the present invention is to provide a pharmaceutical composition, cosmetic composition, or food composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

Additionally, the purpose of the present invention is to provide a pharmaceutical composition, cosmetic composition, or food composition for promoting collagen production, consisting of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparagynamide, prolinamide, glutaminamide, argininamide, threoninamide, valinamide, and tyrosinamide.

Additionally, the objective of the present invention is to provide a pharmaceutical composition, cosmetic composition, or food composition for promoting collagen production, consisting essentially of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

The objective of the present invention is to provide the use of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginemide, prolinamide, glutaminamide, argininamide, threoninamide, valinamide, and tyrosinamide for the preparation of a composition for promoting collagen production.

The objective of the present invention is to provide a method for promoting collagen production, comprising administering to a subject in need thereof an effective amount of a promoting composition comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparagynamide, prolinamide, glutaminamide, argininamide, threoninamide, valinamide, and tyrosinamide.

### MEANS FOR SOLVING THE PROBLEM

To achieve the aforementioned objectives of the present invention, the present invention provides a composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

Furthermore, the present invention provides a composition for promoting collagen production, consisting of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparagynamide, prolinamide, glutaminamide, argininamide, threoninamide, valinamide, and tyrosinamide.

Furthermore, the present invention provides a composition for promoting collagen production, consisting essentially of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparagynamide, prolinamide, glutaminamide, argininamide, threoninamide, valinamide, and tyrosinamide.

To achieve the aforementioned objectives of the present invention, the present invention provides the use of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparagynamide, prolinamide, glutaminamide, argininamide, threoninamide, valinamide, and tyrosinamide for the preparation of a composition for promoting collagen production.

To achieve the aforementioned objectives of the present invention, the present invention provides a method for promoting collagen production, comprising administering to a subject in need thereof an effective amount of a promoting composition comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

Hereinafter, the present invention will be described in detail.

The present invention provides a pharmaceutical composition, cosmetic composition, or food composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

The single-letter (three-letter) abbreviations of amino acids used in this specification consist of the following amino acids according to the standard abbreviation rules in the field of biochemistry:
A(Ala): Alanine; C(Cys): Cysteine; D(Asp): Aspartic acid; E(Glu): Glutamic acid; F(Phe): Phenylalanine; G(Gly): Glycine; H(His): Histidine; I(Ile): Isoleucine; K(Lys): Lysine; L(Leu): Leucine; M(Met): Methionine; N(Asn): Asparagine; O(Ply): Pyrrolysine; P(Pro): Proline; Q(Gln): Glutamine; R(Arg): Arginine; S(Ser): Serine; T(Thr): Threonine; U(Sec): Selenocysteine; V(Val): Valine; W(Trp): Tryptophan; Y(Tyr): Tyrosine.

Additionally, the amino acid sequences of all peptides in this specification are described from the amino terminus (N-terminus, amino terminal, or N-terminal) to the carboxy terminus (C-terminus, carboxy terminal, or C-terminal) in accordance with standard regulations in the field of biochemistry. If an amino group (-NH₂) is indicated at the carboxy terminus of a peptide described in this specification, it does not refer to the amino terminus of the amino acid sequence, but rather indicates that an amino group has been added to the carboxy terminus through carboxy-terminal amidation, a method of chemical synthesis of the peptide.

According to an embodiment of the present invention, alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide promote collagen production in each of human fibroblasts, chondrocytes, myocytes, osteoblasts, follicular cells, and endothelial cells.

Collagen occurs in many parts throughout the body. More than 90 percent of the collagen in the body is Type I. To date, 28 types of collagen have been identified and described. The five most common types of collagen are as follows:
- Collagen I: skin, tendons, vascular ligature, organs, and bones (main component of the organic part of bones);
- Collagen II: cartilage (the main component of cartilage);
- Collagen III: typically found together with Collagen I, the main component of reticulate (reticular fibers);
- Collagen IV: formation of the basal lamina, which is the epithelial secretory layer of the basement membrane; and
- Collagen V: cell surface, hair, and placenta

Collagen is crucial for health, because it plays a key role in maintaining the health of skin, hair, blood vessels, cartilage, tendons, connective tissues, muscles, bones, teeth, and periodontal tissues, as detailed below:
Skin Health: Collagen plays a crucial role in skin health. Collagen I and Collagen III are formed in the skin at higher ratios compared to other types of collagen and are maintained at fixed ratios in normal skin tissue. Collagen I comprises about 70 percent of the collagen in the skin, while Collagen III comprises about 10 percent. Collagen IV, Collagen V, Collagen VI, and Collagen VII each constitute trace amounts of collagen in the skin. Collagen maintains the firmness and elasticity of the skin. Collagen in the form of hydrolyzed collagen helps keep the skin hydrated. The decrease in collagen levels in the body with aging leads to sagging, lines, wrinkles, lack of tension and elasticity, and delayed wound healing.
Wound healing: Collagen is a key protein in connective tissue and plays a crucial role in wound healing and scar formation through repair. Age-related delays in wound healing are caused by impaired collagen production and increased degradation.
Bone: The organic portion of bone comprises about 95% collagen, primarily collagen I. The combination of hard minerals and flexible collagen prevents bones from breaking too easily and makes them harder than cartilage. The mesh of collagen and water forms a strong and slippery pad in the j oint, providing cushioning to the ends of bones within the joint during muscle movement.
Cartilage, Tendons, Ligaments: Elongated fibrillar forms of collagen are primarily found in fibrous tissues, such as tendons and ligaments. This flexible and elastic protein is used by the body to support tissues, thereby playing an essential role in the maintenance of cartilage, tendons, and ligaments. Normal tendons are composed of densely packed bundles of collagen fibers and are surrounded by a tendon sheath, forming a soft and fibrous connective tissue. Collagen II is the main component of cartilage.
Muscle: In muscle tissue, collagen acts as the main component of the endomysium.
Tooth tissue: The organic part of dentin and pulp consists primarily of collagen, mainly collagen I, with small amounts of collagen III and collagen IV The main collagen found in cementum is collagen I, while in the periodontal ligament, it consists of collagen I, collagen III, and collagen XII.
Basement membrane: The epithelial basement membrane consists of collagen IV and collagen VII.

Collagen-related disorders most commonly arise from genetic defects or nutritional deficiencies that affect biosynthesis, assembly, post-translational modification, secretion, or other processes involved in normal collagen production. Various collagen-related disorders are described below:
Osteogenesis imperfecta is a dominant autosomal disorder consisting of mutations in collagen I. Osteogenesis imperfecta results in weak bones and irregular connective tissue. In some cases, it may be mild, but in severe cases, it can be fatal. Mild cases are characterized by reduced levels of collagen I, whereas severe cases are characterized by structural defects in collagen.

Chondrodysplasias, consisting of mutations in collagen II, are skeletal disorders believed to be the subject of ongoing research efforts.

Osteoporosis is experienced with increasing age rather than being inherited genetically, and it is associated with reduced collagen levels in the skin and bones. Growth hormone injections are being studied as a potential treatment for osteoporosis to counteract any loss of collagen.

Dermal papilla cells, which are crucial for hair development and growth, are located at the very bottom of the hair root in the subcutaneous layer of the scalp. In practice, when dermal papilla cells are harvested from the scalp, cultured, and then transplanted, new hair growth can occur. However, there are several challenges in using dermal papilla cells as a treatment for hair loss. Firstly, it is difficult to isolate these cells from the scalp, the culture conditions are stringent, and it is challenging to culture a sufficient quantity of cells. Additionally, when cultured in large quantities, the hair regeneration capability of these cells significantly decreases. Notably, a large amount of collagen is expressed in dermal papilla cells during the growth phase, but collagen expression decreases in these cells during the regression phase. Therefore, promoting collagen production in dermal papilla cells can improve hair loss.

Collagen is also a component of blood vessels and intestines, and its synthesis decreases or deteriorates with age, leading to various diseases. When there is a deficiency of ascorbic acid, collagen is not synthesized properly, causing damage to the blood vessel walls and resulting in scurvy, which is characterized by bleeding throughout the body. If collagen in the blood vessels (blood vessel walls) is deficient or deteriorated, the vessels lose their elasticity, hindering smooth expansion and contraction, which can lead to hypertension. Particularly, when collagen in the arterial walls deteriorates, it causes arteriosclerosis, which can lead to myocardial infarction or cerebral infarction.

Collagen is essential for healing wounds in our body. It is important not only for the suturing of skin injuries but also for the healing of internal wounds. In the case of the stomach, which is a digestive organ constantly exposed to highly acidic gastric juices, a lack of collagen can easily lead to gastritis and make it difficult for stomach wounds to heal.

As one ages, the lack and degradation of collagen accelerate aging in all organs, with a particularly noticeable decrease in skin collagen, which is a major cause of skin aging. The skin is composed of three layers: the epidermis, dermis, and subcutaneous tissue. The dermis is densely populated with collagen, which plays a role in maintaining skin elasticity. Additionally, the stratum corneum and the lipid film protect the skin surface and retain moisture, but these functions weaken with age. Consequently, as people age, their skin becomes dry, rough, and wrinkled. Consuming collagen helps restore skin elasticity and moisture, reducing wrinkles.

A deficiency of collagen in the scalp can lead to hair loss. Although collagen is not a component of hair itself, hair roots extend into the dermis. Therefore, a lack of collagen in the dermis prevents hair from growing healthily. Conversely, an abundance of collagen results in thicker, healthier hair and an increase in the number of hairs.

The main component of articular cartilage consists of collagen. As one ages, the loss of collagen weakens the function of the cartilage, making it unable to absorb shocks when the joint moves. This results in bones rubbing against each other, causing inflammation in the joint, leading to swelling, fluid accumulation, and ultimately degenerative arthritis, which is accompanied by joint deformation and pain.

Collagen metabolism is closely related to the development and health of bones and teeth throughout life. A deficiency in collagen during childhood can limit the development of bones and teeth as well as overall physical growth. As one ages, insufficient calcium absorption makes one more susceptible to osteoporosis, and a lack of collagen is also a significant cause of osteoporosis.

The main component of the periodontal membrane (periodontal ligament) between the teeth and the alveolar bone (jawbone) consists of collagen. A deficiency in collagen weakens the periodontal membrane and gums, leading to the exposure of the tooth roots, attachment of food debris, and proliferation of bacteria, which causes periodontitis.

The composition provided by the present invention, comprising amidated amino acids, enhances the body's ability to produce collagen by upregulating the production of collagen and related proteins, thereby improving the health of skin, hair, blood vessels, cartilage, tendons, connective tissues, muscles, bones, teeth, and periodontal tissues.

More specifically, the composition according to the present invention can promote collagen production in various cells comprising fibroblasts, follicular cells, dermal papilla cells, endothelial cells, chondrocytes, muscle cells, and osteoblasts.

Therefore, the composition of the present invention can be characterized by its use for the prevention or treatment of diseases or lesions selected from the group consisting of skin elasticity reduction and wrinkles due to aging; hypertension and reduced blood flow due to decreased vascular elasticity; decreased ligament elasticity; decreased joint elasticity; elastosis, dermal-epidermal atrophy, dermal atrophic skin diseases due to photoaging; wounds; burns; skin lesions; pressure ulcers; dermal aplasia induced by drug administration; bone and tooth developmental disorders, osteoporosis; periodontal diseases; and hair loss.

The pharmaceutical composition according to the present invention comprises an amidated amino acid, specifically alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide, which can be included either individually or in combination of two or more.

In a preferred embodiment of the present invention, the pharmaceutical composition comprises glycinamide, isoleucinamide, leucinamide, methioninamide, prolinamide, valinamide, and tyrosinamide, either individually or in combination of two or more.

In the most preferred embodiment of the present invention, the pharmaceutical composition may comprise glycinamide.

The amide-form amino acids according to the present invention can be used either in their free form or as pharmaceutically acceptable salts. In the present invention, "pharmaceutically acceptable" means that the substance is physiologically acceptable, does not interfere with the activity of the active ingredient when administered to humans, and does not typically cause allergic reactions or similar responses such as gastrointestinal disturbances or dizziness. The preferred salts are acid addition salts formed by pharmaceutically acceptable free acids, which can be either organic or inorganic acids. The organic acids include, but are not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, p-toluenesulfonic acid, glutamic acid, and aspartic acid. The inorganic acids include, but are not limited to, hydrochloric acid, hydrobromic acid, sulfuric acid, and phosphoric acid.

The pharmaceutical composition according to the present invention, comprising a pharmaceutically acceptable carrier for promoting collagen production, can be formulated in various ways depending on the route of administration by methods known in the art. The carrier includes all types of solvents, dispersion media, oil-in-water or water-in-oil emulsions, aqueous compositions, liposomes, microbeads, and microsomes.

The pharmaceutical composition according to the present invention can be administered to a patient in an amount effective to promote collagen production. For example, a typical daily dosage can range from about 0.01 to 1000 mg/kg, and preferably from about 50 to 500 mg/kg. The pharmaceutical composition of the present invention can be administered in a single dose or in divided doses within the preferred dosage range. Additionally, the dosage of the pharmaceutical composition according to the present invention can be appropriately selected by a person skilled in the art based on the route of administration, the subject of administration, age, gender, body weight, individual differences, and disease condition.

The administration route can be oral or parenteral. Parenteral administration methods, comprising but not limited to intravenous, intramuscular, intra-arterial, intraosseous, intrathecal, intracardiac, transdermal, subcutaneous, intraperitoneal, intranasal, intestinal, topical, sublingual, or rectal administration.

When the pharmaceutical composition of the present invention is administered orally, it can be formulated into forms such as powders, granules, tablets, pills, sugar-coated tablets, capsules, liquids, gels, syrups, suspensions, and wafers, in accordance with methods known in the art, along with suitable oral administration carriers. Examples of suitable carriers include saccharides comprising lactose, dextrose, sucrose, sorbitol, mannitol, xylitol, erythritol, and maltitol; starches comprising corn starch, wheat starch, rice starch, and potato starch; celluloses comprising cellulose, methyl cellulose, sodium carboxymethyl cellulose, and hydroxypropyl methyl cellulose; and fillers such as gelatin and polyvinylpyrrolidone. Additionally, cross-linked polyvinylpyrrolidone, agar, alginic acid, or sodium alginate may be added as disintegrants, as needed. Furthermore, the pharmaceutical composition may additionally comprise anti-agglomerating agents, lubricants, humectants, flavoring agents, emulsifiers, and preservatives.

Additionally, when administered parenterally, the pharmaceutical composition of the present invention can be formulated into forms such as injectables, transdermal delivery systems, and nasal inhalants using methods well-known in the art, along with suitable parenteral carriers. In the case of injectables, they must be sterile and protected from contamination by microorganisms such as bacteria and fungi. Suitable carriers for injectables may include, but are not limited to, solvents or dispersion media comprising water, ethanol, polyols (e.g., glycerol, propylene glycol, and liquid polyethylene glycol), mixtures thereof, and/or vegetable oils. More preferably, suitable carriers may include isotonic solutions such as Hank's solution, Ringer's solution, phosphate-buffered saline (PBS) containing triethanolamine, or sterile water for injection, 10% ethanol, 40% propylene glycol, and 5% dextrose. To protect the injectables from microbial contamination, various antibacterial and antifungal agents such as parabens, chlorobutanol, phenol, sorbic acid, and thimerosal may be additionally included. Furthermore, the injectables may also include isotonic agents such as sugars or sodium chloride in most cases.

In the case of transdermal administration, forms such as ointments, creams, lotions, gels, external solutions, pastes, liniments, and aerosols are included. "transdermal administration" means delivering an effective amount of the active ingredient contained in the pharmaceutical composition into the skin by topically applying the pharmaceutical composition to the skin. For example, the pharmaceutical composition of the present invention can be prepared in an injectable form and administered by lightly pricking the skin with a fine 30-gauge needle or by directly applying it to the skin. These formulations are described in prescriptions generally known in pharmaceutical chemistry.

In the case of inhalation administration, the compound used according to the present invention can be conveniently delivered in the form of an aerosol spray from a pressurized pack or nebulizer using a suitable propellant, such as dichlorofluoromethane, trichlorofluoromethane, dichlorotetrafluoroethane, carbon dioxide, or another suitable gas. For pressurized aerosols, the dosage unit can be determined by providing a valve that delivers a metered amount. For example, gelatin capsules and cartridges used in inhalers or insufflators can be formulated to contain a powder mixture of the compound and a suitable powder base such as lactose or starch.

Other pharmaceutically acceptable carriers can be referenced from those known in the art.

Additionally, the pharmaceutical composition according to the present invention may further comprise one or more buffering agents (e.g., saline or PBS), carbohydrates (e.g., glucose, mannose, sucrose, or dextran), antioxidants, bacteriostatic agents, chelating agents (e.g., EDTA or glutathione), adjuvants (e.g., aluminum hydroxide), suspending agents, thickeners, and/or preservatives.

Furthermore, the pharmaceutical composition of the present invention can be formulated using methods known in the art to provide rapid, sustained, or delayed release of the active ingredient after administration to a mammal.

Additionally, the pharmaceutical composition of the present invention can be administered alone or in combination with known compounds that have a collagen production-promoting effect.

In one aspect of the present invention, the known compound may comprise ascorbic acid, asiaticoside, glucosamine, or their analogs and derivatives.

In the present invention, the known compound can be administered in combination with the amidated amino acid in a molar ratio of amidated amino acid:known compound = 1:0.1 to 5.

In one embodiment of the present invention, it has been confirmed that the combined treatment of amidated amino acids with ascorbic acid, asiaticoside, glucosamine, or their analogs results in a synergistic or cooperative effect in promoting intracellular collagen production.

In the present invention, the ascorbic acid analogs may be selected from the group consisting of magnesium ascorbyl phosphate, 3-O-ethyl ascorbic acid, ascorbyl glucoside, and ascorbyl tetraisopalmitate.

The present invention also provides a cosmetic composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

The cosmetic composition may be used for skin wrinkle improvement, skin moisture enhancement, skin elasticity improvement, or skin aging prevention.

The cosmetic composition according to the present invention may comprise one amidated amino acid selected from the group, or it may comprise a combination of two or more amidated amino acids selected from the group.

The cosmetic composition of the present invention can be manufactured in any formulation commonly produced in the art, and in addition to the amidated amino acid, it can be formulated as an adjuvant for topical or systemic application commonly used in the dermatological field by comprising a dermatologically acceptable medium or base.

Additionally, the cosmetic composition of the present invention may further comprise, in addition to the amidated peptide, auxiliary agents commonly used in the fields of cosmetology or dermatology, such as fatty substances, organic solvents, solubilizers, thickeners and gelling agents, emollients, antioxidants, suspending agents, stabilizers, foaming agents, fragrances, surfactants, water, ionic or non-ionic emulsifiers, fillers, metal ion sequestering agents and chelating agents, preservatives, vitamins, blockers, humectants, essential oils, dyes, pigments, hydrophilic or lipophilic active agents, lipid vesicles, or any other ingredients conventionally used in cosmetics. Furthermore, these components can be introduced in amounts generally used in the field of dermatology.

Suitable formulations of the cosmetic composition may include, for example, solutions, gels, solid or paste anhydrous products, emulsions obtained by dispersing an oil phase in an aqueous phase, suspensions, microemulsions, microcapsules, microgranules, or vesicular dispersions in ionic (liposome) or non-ionic forms, creams, skins, lotions, powders, ointments, sprays, or conceal sticks. Additionally, it can also be manufactured in the form of foams or aerosol compositions further comprising compressed propellants. Products to which the cosmetic composition of the present invention can be added include, but are not limited to, skin lotions, essences, nutritional essences, packs, soaps, shampoos, cleansing foams, cleansing lotions, cleansing creams, body lotions, body cleansers, treatments, beauty liquids, emulsions, pressed powders, loose powders, and eye shadows.

The content of the amide-modified amino acid contained in the cosmetic composition of the present invention comprises 0.0001 to 50% by weight, preferably 0.01 to 10% by weight, based on the total weight of the cosmetic composition, which can be appropriately determined by a person skilled in the art considering factors such as the desired effect, application extent, type of formulation, and stability of the peptide within the cosmetic composition.

The present invention also provides a food composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

The food composition may be characterized by its use for the prevention or improvement of conditions or lesions selected from the group consisting of skin elasticity reduction and wrinkles due to aging; hypertension and poor blood circulation due to decreased vascular elasticity; decreased ligament elasticity; decreased joint elasticity; elastosis, dermal-epidermal atrophy, dermal atrophic skin diseases due to photoaging; wounds; burns; skin lesions; pressure ulcers; dermal aplasia induced by drug administration; bone and tooth developmental disorders, osteoporosis; periodontal diseases; and hair loss.

The food composition according to the present invention comprises all forms such as functional food, nutritional supplement, health food, and food additives. These types can be manufactured in various forms according to conventional methods known in the art. To use the food composition of the present invention in the form of a food additive, it can be prepared and used in the form of powder or concentrate.

Meanwhile, the food composition according to the present invention may comprise one of the amidated amino acid selected the group, or may comprise a combination of two or more amidated amino acids selected the group.

As a health food, the food composition of the present invention can be consumed by preparing it in the form of tea, juice, or drink, or by granulating, encapsulating, or powdering it. Additionally, the food composition of the present invention can be formulated in combination with known substances or active ingredients that are known to promote collagen production.

The functional foods can be manufactured by adding the food composition of the present invention to beverages (including alcoholic beverages), fruits and their processed products (e.g., canned fruits, bottled fruits, jams, marmalades, etc.), fish, meat and their processed products (e.g., ham, sausage, corned beef, etc.), bread and noodles (e.g., udon, buckwheat noodles, ramen, spaghetti, macaroni, etc.), fruit juices, various drinks, cookies, taffy, dairy products (e.g., butter, cheese, etc.), edible vegetable oils, margarine, vegetable proteins, retort foods, frozen foods, and various seasonings (e.g., soybean paste, soy sauce, sauces, etc.).

The preferred content of the peptide in the food composition according to the present invention, but is not limited thereto, is preferably 0.01 to 50% by weight in the finally manufactured food.

The present invention provides the use of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide for the preparation of a composition for promoting collagen production.

The present invention provides a method for promoting collagen production, comprising administering to a subject in need thereof an effective amount of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginamide, prolinamide, glutaminamide, argininamide, threoninamide, valinamide, and tyrosinamide.

The term "effective amount" in the present invention refers to an amount that, when administered to a subject, exhibits an effect of promoting collagen production, wherein the "subject" can be an animal, preferably a mammal, particularly an animal including a human, and may also be cells, tissues, organs, etc., derived from animals. The subject may be a patient in need of the aforementioned effect.

In this specification, the term "comprising" is used in the same meaning as "including" or "characterized by," and in the context of the composition or method according to the present invention, it does not exclude additional components or method steps that are not specifically mentioned. Additionally, the term "consisting of" means excluding any additional elements, steps, or components not explicitly stated. The term "consisting essentially of" means that the composition or method may include substances or steps that do not materially affect the basic characteristics of the listed substances or steps.

### EFFECTS OF THE INVENTION

The composition of the present invention, comprising amidated amino acids, can be very useful in the development of treatments for various diseases and lesions that may arise due to collagen deficiency by promoting collagen production within cells.

### BRIEF DESCRIPTION OF THE DRAWINGS

FIGs. 1a and 1b show the results of evaluating the effects of 20 amidated amino acids on collagen production and cell viability in human dermal fibroblasts.
FIGs. 2a and 2b show the results of evaluating the effects of 20 free amino acids on collagen production and cell viability in human dermal fibroblasts.
FIGs. 3a to 3c show the results of evaluating the effects of glycine or its analogs on collagen production and cell viability in human dermal fibroblasts.
FIGs. 4a to 4d show the concentration-dependent effects of ascorbic acid (AA), glycine, and glycinamide on collagen production and cell viability in human dermal fibroblasts.
FIGs. FIGs. 5a and 5b show the results evaluating the effects of individual or combined treatments of Ascorbic Acid (AA), Glycinamide, and TGF-β1 on collagen production and cell proliferation in human dermal fibroblasts.
FIGs. 6a and 6b show the results of evaluating the effects of individual or combined treatments of Ascorbic Acid (AA), Glycinamide, and TGF-β1 on wound healing in human dermal fibroblasts.
FIGs. 7a to 7c show the results of evaluating the effects of individual or combined treatments of ascorbic acid (AA), its analogs, glycinamide, and TGF-β1 on collagen production and cell proliferation in human dermal fibroblasts.
FIGs. 8a and 8b show the results of evaluating the effects of individual or combined treatments of ascorbic acid (AA), its analogs, glycinamide, and TGF-β1 on wound healing in human dermal fibroblasts.
FIGs. 9a and 9b show the results of evaluating the effects of asiaticoside, madecassoside, asiatic acid, and madecassic acid on cell viability and collagen production in human dermal fibroblasts.
FIGs. 10a and 10b show the results evaluating the effects of asiaticoside, madecassoside, and ascorbic acid on cell viability and collagen production in human dermal fibroblasts.
FIGs. 11a and 11b show the results evaluating the effects of individual or combined treatments of Asiaticoside and Glycinamide on the viability and collagen production of human dermal fibroblasts.
FIGs. 12a to 12j show the results of evaluating the effects of glycine (G), proline (P), lysine (K), or their amidated amino acids (G-NH₂, P-NH₂, K-NH₂) on collagen production and cell proliferation (MTT reduction) in human articular chondrocytes (HAC), human dermal fibroblasts (HDF), human follicle dermal papilla cells (HFCPC), human aortic endothelial cells (HAoEC), and human osteoblasts (HOB).
FIGs. 13a to 13l show the concentration-dependent effects of Glucosamine (GlcN), Glycinamide (G-NH₂), and TGF-β1 treatments on collagen production and cell proliferation (MTT reduction) in human dermal fibroblasts and human articular chondrocytes.
FIGs. 14a to 14d show the results evaluating the effects of individual or combined treatments of Glucosamine (GlcN) and Glycinamide (G-NH₂) on collagen production and cell proliferation (MTT reduction) in human articular chondrocytes and human dermal fibroblasts.

### DETAILED DESCRIPTION FOR IMPLEMENTING THE INVENTION

Hereinafter, the present invention will be described in detail by the following embodiments. However, the following embodiments are merely illustrative of the present invention and the present invention is not limited thereto.

### Experimental Method

### 1. Experimental sample

In this study, 20 types of free amino acids and 20 types of amidated amino acids were used. The compounds are listed in Table 1 using single-letter codes for the amino acids. The free amino acids were purchased from Sigma-Aldrich (St. Louis, MO, USA). The amidated amino acids were purchased from Watanabe Chemical Ind., Ltd. (Hiroshima, Japan). N-acetyl glycine, N-acetyl glycinamide, glycine methyl ester HCl, glycine ethyl ester HCl, glycyl glycine, ascorbic acid (AA), 3-O-ethyl ascorbic acid (EAA), and ascorbyl 2-O-glucoside (AG), glucosamine HCl were purchased from Sigma-Aldrich. Magnesium ascorbyl phosphate (MAP) and ascorbyl tetraisopalmitate (ATI) were purchased from Biosynth Carbosynth (Berkshire, UK). Recombinant human TGF-β1 protein was purchased from R&D Systems (Minneapolis, MN, USA). Asiaticoside, madecassoside, asiatic acid, and madecassic acid were purchased from MedChemExpress (Monmouth Junction, NJ, USA).

**[Table 1]**

| Free Amino Acids | Single-Letter Codes | Amidated Amino Acids | Single-Letter Codes |
|---|---|---|---|
| L-Alanine | A | L-Alaninamide HCl | A-NH₂ |
| L-Cysteine | C | L-Cysteinamide HCl | C-NH₂ |
| L-Aspartic acid | D | L-Aspartic acid α-amide HCl | D-NH₂ |
| L-Glutamic acid | E | L-Glutamic acid α-amide | E-NH₂ |
| L-Phenylalanine | F | L-Phenylalaninamide HCl | F-NH₂ |
| Glycine | G | Glycinamide HCl | G-NH₂ |
| L-Histidine | H | L-Histidinamide HCl | H-NH₂ |
| L-Isoleucine | I | L-Isoleucinamide HCl | I-NH₂ |
| L-Lysine | K | L-Lysinamide diHCl | K-NH₂ |
| L-Leucine | L | L-Leucinamide HCl | L-NH₂ |
| L-Methionine | M | L-Methioninamide HCl | M-NH₂ |
| L-Asparagine | N | L-Asparaginamide HCl | N-NH₂ |
| L-Proline | P | L-Prolinamide HCl | P-NH₂ |
| L-Glutamine | Q | L-Glutaminamide HCl | Q-NH₂ |
| L-Arginine | R | L-Argininamide diHCl | R-NH₂ |
| L-Serine | S | L-Serinamide HCl | S-NH₂ |
| L-Threonine | T | L-Threoninamide HCl | T-NH₂ |
| L-Valine | V | L-Valinamide HCl | V-NH₂ |
| L-Tryptophane | W | L-Tryptophanamide HCl | W-NH₂ |
| L-Tyrosine | Y | L-Tyrosinamide HCl | Y-NH₂ |

### 2. Cell culture

Human dermal fibroblasts (HDF) extracted from adult skin were obtained from Cascade Biologics (Portland, OR, USA), and human articular chondrocytes (HAC), human follicle dermal papilla cells (HFCPC), human aortic endothelial cells (HAoEC), and human osteoblasts (HOB) were obtained from PromoCell (Heidelberg, Germany). The cells were cultured in a closed incubator at 37°C in humidified air comprising 5% CO₂. The growth medium for HDF consisted of Iscove's Modified Dulbecco's Medium (IMDM) (Gibco BRL, Grand Island, NY, USA) supplemented with 10% fetal bovine serum (Hyclone, South Logan, UT) and 1% antibiotics (100 U·mL⁻¹ penicillin, 0.1 mg·mL⁻¹ streptomycin). HAC were cultured in Chondrocyte Growth Medium, HFCPC in Follicle Dermal Papilla Cell Growth Medium, HAoEC in Endothelial Cell Medium MV, and HOB in Osteoblast Growth Medium (PromoCell, Heidelberg, Germany), all supplemented with 10% fetal bovine serum (Hyclone, South Logan, UT) and 1% antibiotics (100 U·mL⁻¹ penicillin, 0.1 mg·mL⁻¹ streptomycin).

### 3. Cell viability assay

The cell viability was evaluated using the MTT assay. Briefly, HDF (1 × 10⁴cells per well), HAC (5 × 10³cells per well), HFDPC (1 × 10⁴cells per well), HAoEC (5 × 10³cells per well), and HOB (2 × 10³cells per well) were cultured in growth media in a 96-well culture plate for 24 hours, followed by treatment with each drug for 48 hours. After discarding the media, the cells were incubated at 37°C for 2 hours in fresh growth media (100 µL) containing 1 mg·mL⁻¹ MTT (Amresco, Solon, OH, USA). The media was removed by aspiration, and the formazan dye accumulated inside the cells was dissolved in 100 µL of dimethyl sulfoxide. The absorbance of the solution was measured at 570 nm using a Spctrostar Nano microplate reader (BMG LABTECH GmbH, Ortenberg, Germany).

### 4. Measurement of collagen protein

The collagen protein levels in the conditioned media were evaluated using the Sircol^{™} Collagen Assay (Biocolor, Antrim, UK) according to the manufacturer's instructions. HDF (2 × 10⁵cells per well), HAC (3 × 10⁵cells per well), HFDPC (2 × 10⁵cells per well), HAoEC (3 × 10⁵cells per well), and HOB (3 × 10⁵cells per well) were cultured in growth media in 12-well culture plates for 24 hours, followed by treatment with each drug for an additional 24 hours. After discarding the media, the cells were washed with 500 µL of phosphate-buffered saline (PBS), then incubated in 1 mL of serum-free media for another 24 hours. A 1.0 mL media sample (either conditioned media or fresh media as a negative control) was transferred to a microcentrifuge tube, mixed with 100 µL of collagen isolation and concentration reagent, and incubated overnight at 4°C. The samples were centrifuged at 13,000 × g for 10 minutes at 4°C, and the supernatant was carefully removed using a pipette without disturbing the pellet. Each tube was then added with 500 µL of Sircol dye reagent, inverted five times, and shaken at room temperature for 30 minutes. After centrifugation, the supernatant was discarded, and the pellet was washed with 500 µL of cold acid-salt washing reagent, followed by another centrifugation step. The pellet was then dissolved in 250 µL of alkali reagent, and a 200 µL portion was transferred to a 96-well plate. The optical density of the solution was read at 555 nm using a Spectrostar Nano microplate reader (BMG LABTECH GmbH, Ortenberg, Germany).

### 5. Wound healing assay

Wound closure analysis was performed using the Oris^{™} Universal Cell Migration Assembly kit (Platinus Technologies, WI, USA) according to the manufacturer's instructions. The Oris^{™} stoppers were inserted into each well of the Oris^{™}-compatible 96-well plate, and the plate was inverted to check for complete stopper attachment. A suspension of human dermal fibroblasts in IMDM was added to each well (1 × 10⁴cells in 100 µL per well) and incubated for 12 hours to allow cell attachment. Then, the stoppers were removed using the stopper tool, and the medium was aspirated using a pipette. The wells were gently washed with 100 µL PBS to remove unattached cells, and the attached cells were treated with drugs in 100 µL culture medium for the specified time, respectively. Images of the wounds were obtained using a phase-contrast microscope (Eclipse TS100, Nikon Instruments Inc., Melville, NY, USA). The wound areas in the images were quantified using the NIH Image J program. Wound closure (%) was calculated using the following equation: Wound closure (%) = [(A0-At)/A0] × 100, where A0 is the area of the original wound and At is the wound area measured at the specified time.

### Experimental results

### 1. The effect of amidated amino acids on collagen production in human dermal fibroblasts

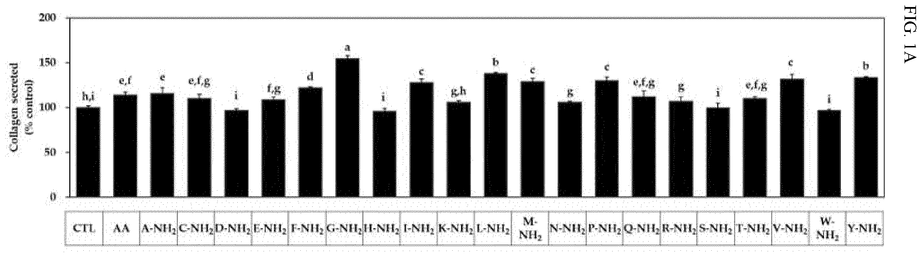

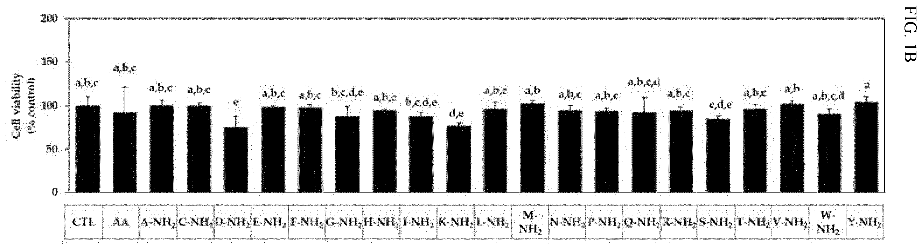

In the first experiment, all 20 types of C-terminal amidated amino acids, which correspond to naturally occurring free amino acids, were compared with ascorbic acid. Cells were treated with each amidated amino acid or ascorbic acid at 1 mM. After 48 hours, the conditioned media were harvested and used for collagen protein quantification by colorimetric assay. Adherent cells were used to assess cell viability using MTT. As shown in Figure 1a, various types of amino acids enhanced the collagen levels secreted into the media, with glycinamide (G-NH₂) being the most effective, followed by leucinamide (L-NH₂), tyrosinamide (Y-NH₂), valinamide (V-NH₂), prolinamide (P-NH₂), methioninamide (M-NH₂), isoleucinamide (I-NH₂), phenylalaninamide (F-NH₂), alaninamide (A-NH₂), glutaminamide (Q-NH₂), cysteinamide (C-NH₂), threoninamide (T-NH₂), glutamic acid α-amide (E-NH₂), and argininamide (R-NH₂). However, other amidated amino acids such as asparaginamide (N-NH₂), lysinamide (K-NH₂), serinamide (S-NH₂), tryptophanamid (W-NH₂), aspartic acid α-amide (D-NH₂), and histidinamide (H-NH₂) had no significant effect. The impact on cell viability, as shown in Figure 1b, indicated that lysinamide and aspartic acid α-amide had a reducing effect, while other amidated amino acids had no significant effect. Therefore, glycinamide was identified as the most effective in enhancing cellular collagen production.

### 2. The effect of free amino acids on collagen production in human dermal fibroblasts.

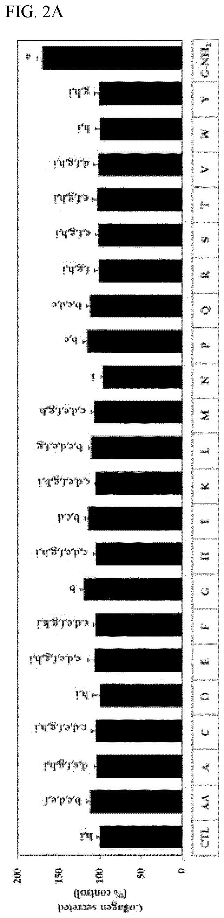

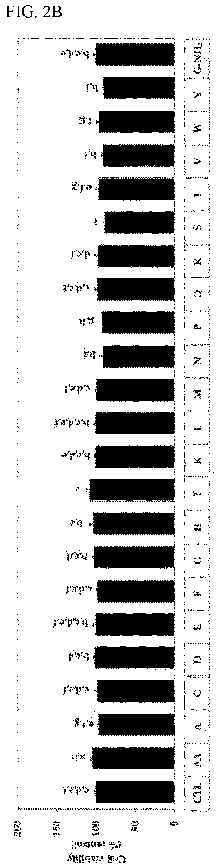

In the following experiment, all 20 naturally occurring free amino acids were compared with ascorbic acid. Cells were treated with each amino acid or ascorbic acid at 1 mM for 48 hours. As shown in Figure 2a, among the free amino acids, glycine (G) most effectively increased collagen, followed by proline (P), isoleucine (I), glutamine (Q), and leucine (L), while other amino acids did not increase collagen. In this experiment, glycylamine (G-NH₂) was also compared and was found to have a superior collagen-promoting effect compared to all free amino acids. In the cell viability experiment, various amino acids had a minimal impact on cell viability (Figure 2b).

### 3. The effect of glycine or its analogs on collagen production in human dermal fibroblasts.

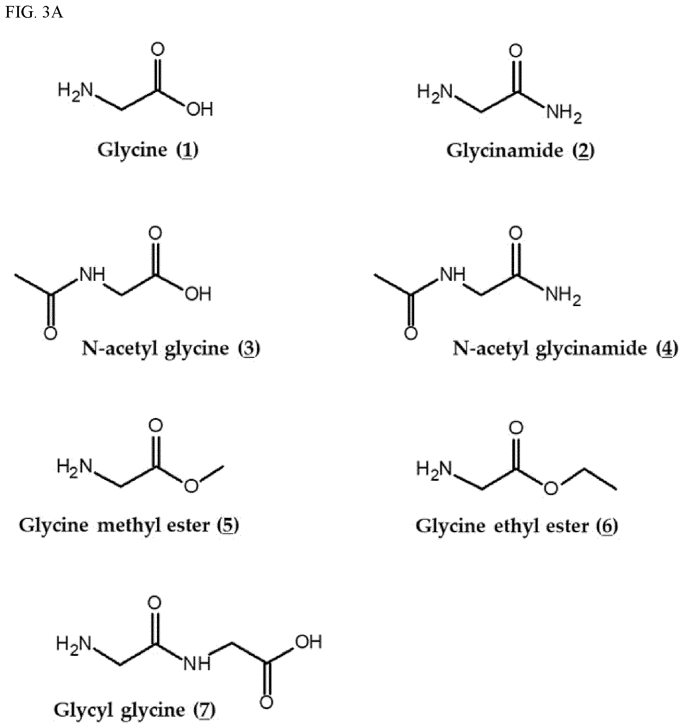

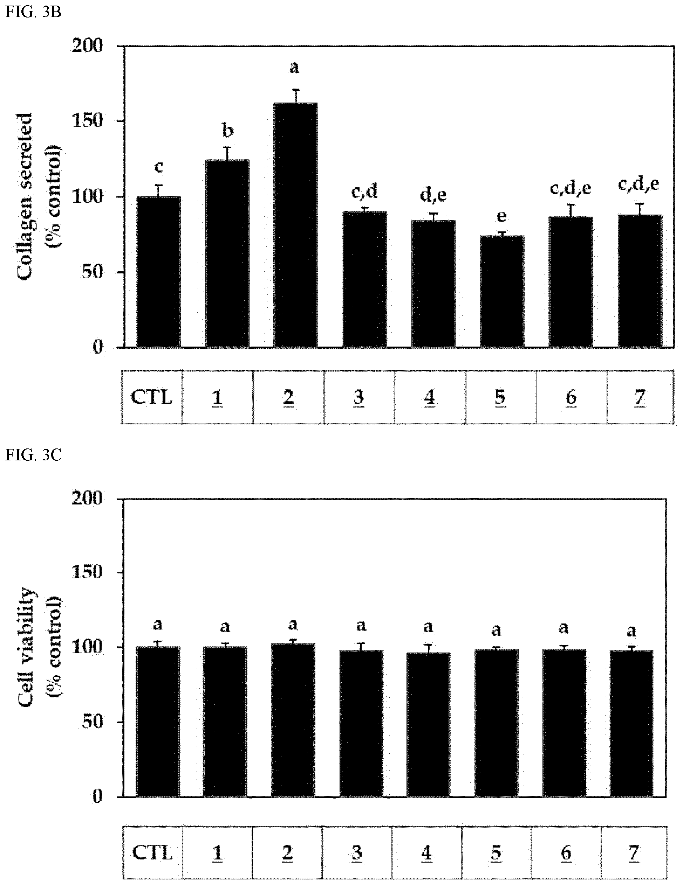

In the following experiment, the effects of various glycine analogs (Fig. 3a) on cellular collagen production were compared. When cells were treated with each glycine analog at a concentration of 1 mM, glycinamide showed the highest collagen production-promoting effect, which was significantly improved compared to glycine (Fig. 3b). While other glycine analogs such as N-acetyl glycine, glycine ethyl ester, and glycyl glycine did not affect collagen production, N-acetyl glycinamide and glycine methyl ester had a somewhat reducing effect. All glycine analogs did not significantly affect cell viability (Fig. 3c).

### 4. Concentration-dependent effects comprising ascorbic acid, glycine, and glycinamide

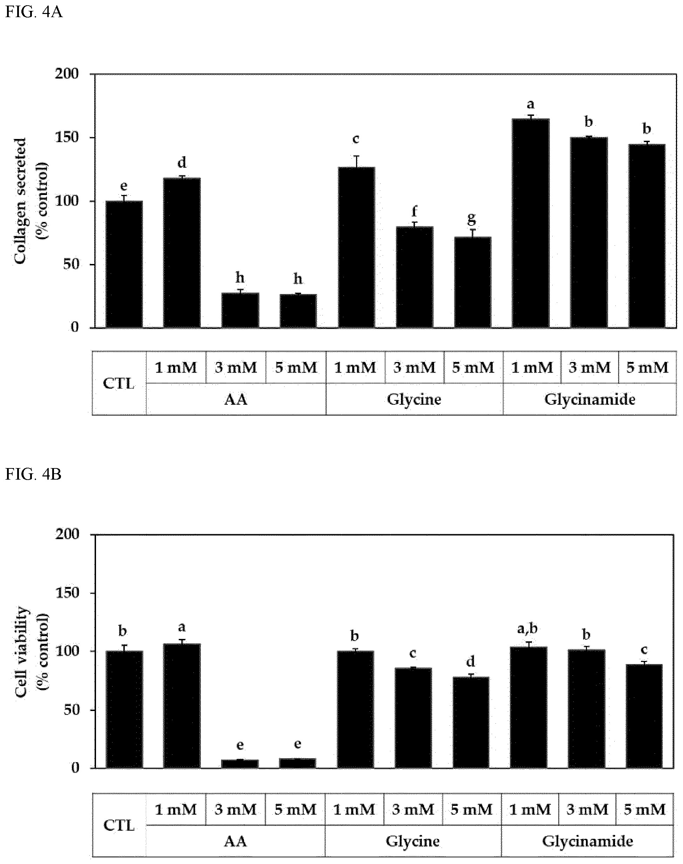

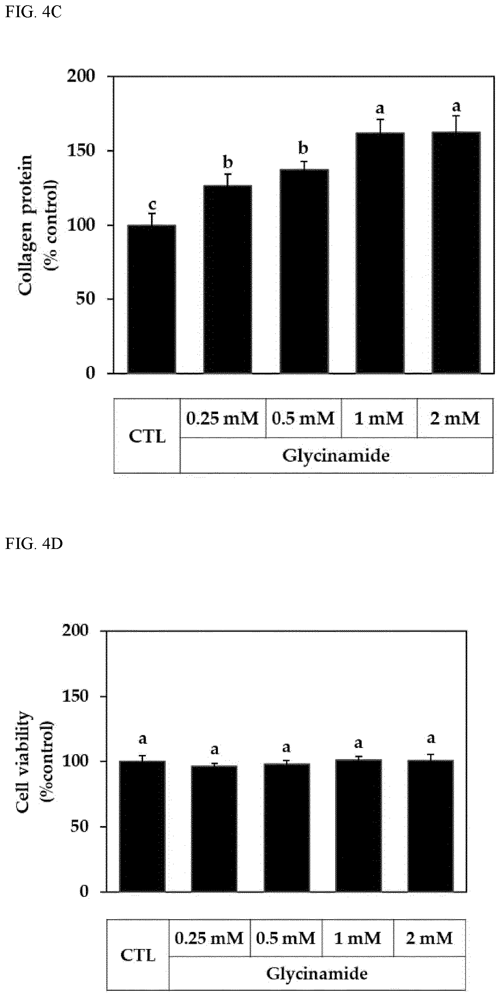

In the following experiment, the effects of ascorbic acid, glycine, and glycylglycine on collagen production were compared based on their concentrations. As shown in Figures 4a and 4b, ascorbic acid increased collagen production at 1 mM without affecting cell viability, but significantly reduced cell viability and collagen production at 3 and 5 mM. Glycine at 1 mM enhanced collagen production without affecting cell viability, but at 3 or 5 mM, it significantly reduced both cell viability and collagen production. Glycylglycine at 1 mM promoted collagen production without affecting cell viability, but at 3 or 5 mM, it slightly reduced cell viability and collagen production. As shown in Figures 4c and 4d, glycylglycine increased collagen production even at a low concentration of 0.25 mM and exhibited the greatest enhancement of collagen production at 1-2 mM without reducing cell viability.

### 5. The effect of individual or combined treatment with ascorbic acid, glycinamide, and TGF-β1 on collagen production in human dermal fibroblasts.

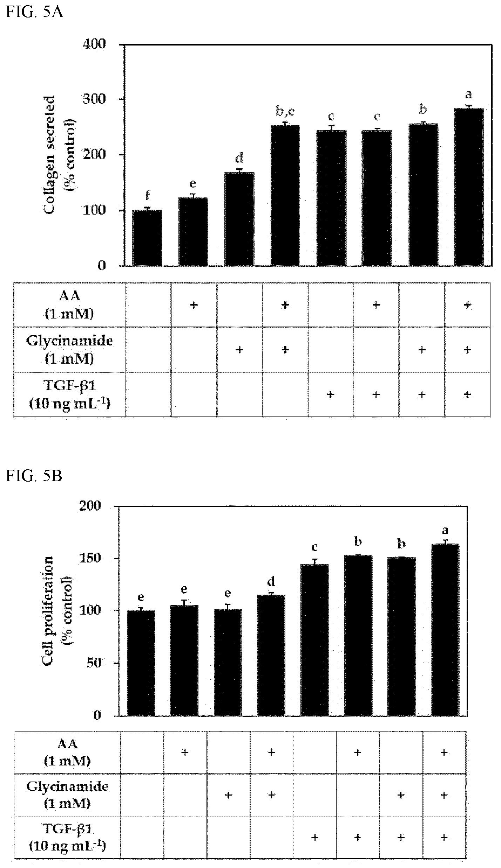

Since it was observed that ascorbic acid and glycinamide each promote collagen production, further investigation was conducted to determine whether their combined treatment exhibits a synergistic effect in the absence and presence of TGF-β1. As shown in Figure 5a, in the absence of TGF-β1, ascorbic acid (1 mM) and glycinamide (1 mM) increased collagen production by 23.1% and 68.2%, respectively, compared to the control. Their combined treatment increased collagen production by 153% compared to the control, which is significantly higher than the arithmetic sum of the individual effects (91.3%). This experimental result demonstrated that the combined treatment of ascorbic acid and glycinamide synergistically promotes collagen production. TGF-β1 (10 ng mL⁻¹) itself, as expected, showed an effect of enhancing collagen production. The combined treatment of ascorbic acid and glycinamide increased collagen production to a similar extent as TGF-β1. The enhancement effect of collagen production by individual or combined treatment of ascorbic acid and glycinamide was greater in the absence of TGF-β1 than in its presence. As shown in Figure 5b, TGF-β1 significantly increased the number of viable cells by 44.5% compared to the control. When ascorbic acid and glycinamide were individually treated, there was no significant change in cell viability; however, their combined treatment significantly increased the number of viable cells by 14.8% compared to the control. This result also demonstrates that the combined treatment of ascorbic acid and glycinamide synergistically promotes cell proliferation.

### 6. The effects of ascorbic acid, glycinamide, and TGF-β1 on wound healing in human dermal fibroblasts.

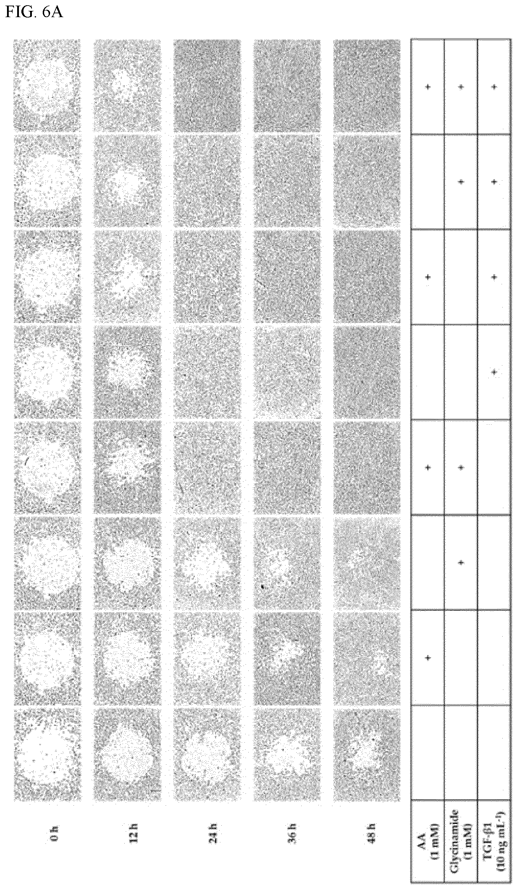

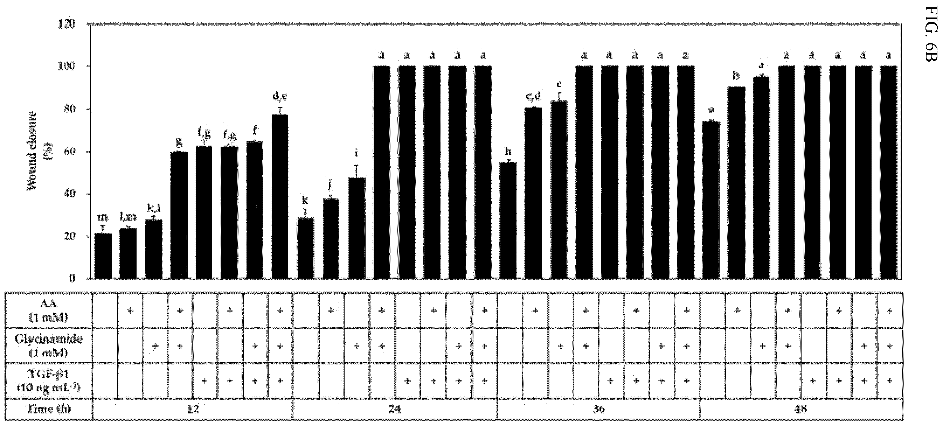

In the following experiment, it was investigated whether individual or combined treatment with ascorbic acid, glycinamide, and TGF-β1 could promote wound closure. Cells were cultured in wells with stoppers to create circular wounds. After removing the stoppers, the cells were treated with each drug for up to 48 hours to monitor wound closure over time. Representative microscopic images of the wounds before and after treatment are shown in Figure 6a. At the start, the extent of the wounds was the same across all groups, but the degree of wound closure varied depending on the treatment. Relative wound closure was quantified through image analysis, and the results are shown in Figure 6b. After 12 hours of treatment, ascorbic acid or glycinamide significantly improved wound closure compared to the control group, and the combined treatment further increased the effect, reaching a level similar to the TGF-β1 treatment group. The change induced by the combined treatment of ascorbic acid and glycinamide (32.1%) was much greater than the arithmetic sum of the changes induced by individual treatments (2.5% + 6.3% = 8.8%). These experimental results suggest that the combined treatment of ascorbic acid and glycinamide can synergistically promote wound closure. The synergistic effect of the combined treatment of ascorbic acid and glycinamide was also observed 24 hours after treatment (100% >> 9.1% + 19.2%). Even in the presence of TGF-β1, the combined treatment of ascorbic acid and glycinamide further enhanced wound closure. These results suggest that the combined treatment of ascorbic acid and glycinamide is as effective as TGF-β1 in promoting wound closure.

### 7. The effects of various ascorbic acid analogs, glycinamide, and TGF-β1 on collagen production in human dermal fibroblasts.

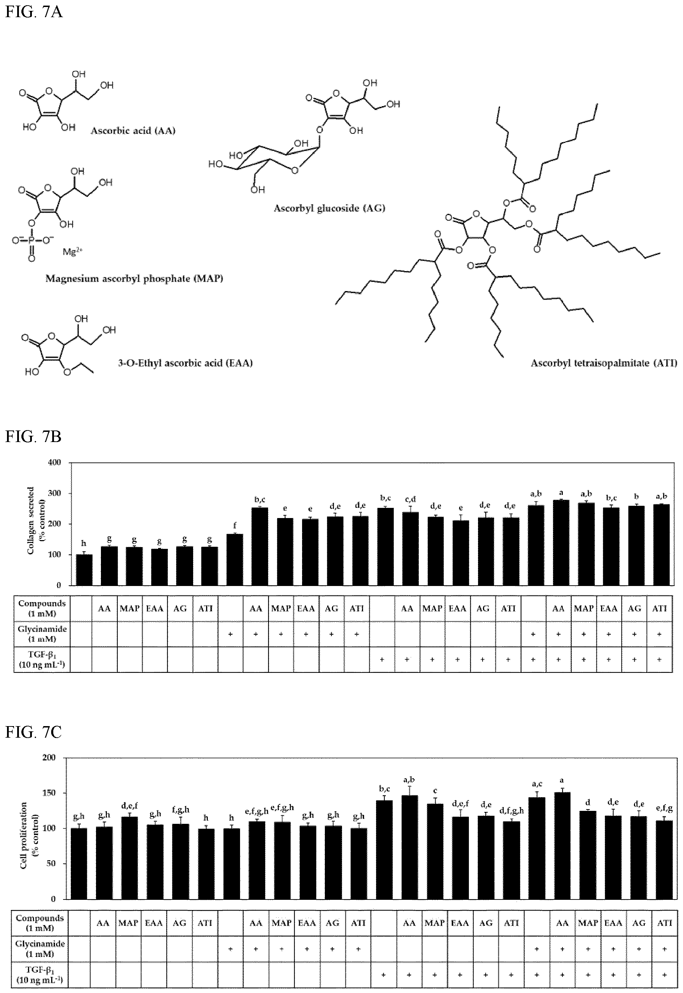

To investigate the effects of various ascorbic acid analogs on collagen production (Fig. 7a), cells were treated with each analog alone or in combination with glycinamide and/or TGF-β1, and collagen production and cell viability were assessed. Ascorbic acid analogs such as magnesium ascorbyl phosphate (MAP), 3-O-ethyl ascorbic acid (EAA), ascorbyl 2-O-glucoside (AG), and ascorbyl tetraisopalmitate (ATI) significantly enhanced collagen production to a similar extent as ascorbic acid (Fig. 7b). When each ascorbic acid analog was combined with glycinamide, a synergistic effect on collagen production was observed. The changes induced by the combined treatment of ascorbic acid analogs and glycinamide (MAP, 118.4%; EAA, 115.5%; AG, 123.6%; ATI, 124.6%) were greater than the arithmetic sums of the changes induced by individual treatments of ascorbic acid analogs and glycinamide (MAP, 25.3% + 67.4% = 92.7%, EAA, 19.5% + 67.4% = 86.9%, AG, 27.2% + 67.4% = 94.6%, ATI, 25.5% + 67.4% = 92.9%). These experimental results demonstrate that the combined treatment of ascorbic acid analogs and glycinamide can synergistically promote collagen production. These results suggest that specific ascorbic acid analogs may be used in combination with glycinamide to enhance collagen production. Cell viability was not significantly affected when treated with each ascorbic acid analog alone or in combination with glycinamide; however, some ascorbic acid analogs (EAA, AG, and ATI) significantly reduced the cell proliferation effect of TGF-β1 (Fig. 7c).

### 8. The effects of various ascorbic acid analogs, glycinamide, and TGF-β1 on wound healing in human dermal fibroblasts.

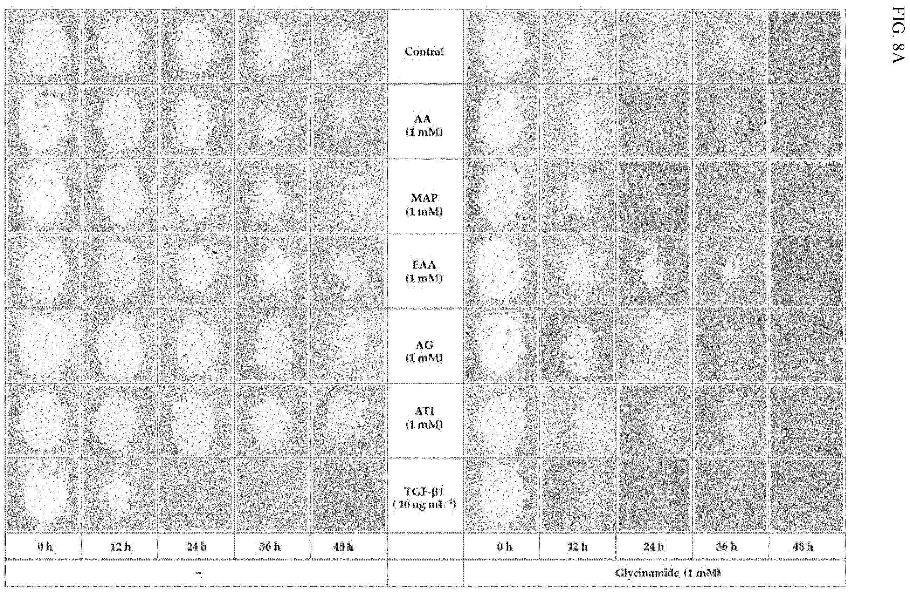

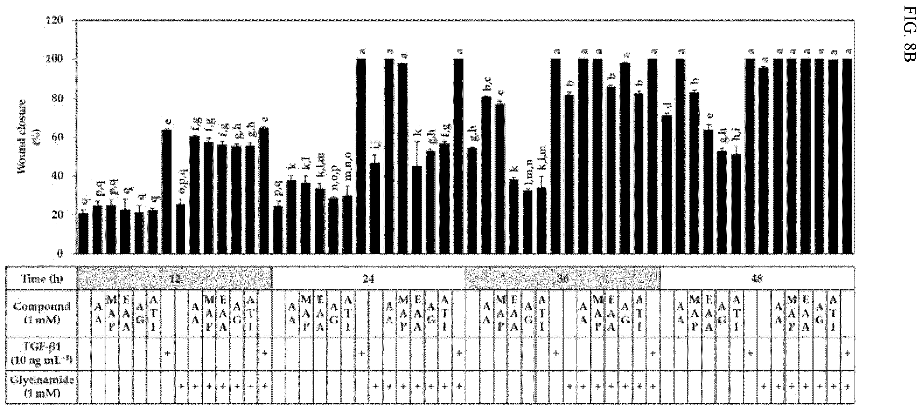

The effects of various ascorbic acid analogs on wound closure were compared in the absence or presence of glycinamide. TGF-β1 was tested for comparison purposes. To monitor wound closure over time, cells were treated with vehicle or drugs for 48 hours. At the start, the extent of wounds in all groups was the same, but the degree of wound closure varied depending on the treatment (Fig. 8a). Relative wound closure was quantified through image analysis, and the results are shown in Fig. 8b. TGF-β1 significantly improved wound closure compared to the control at 12 and 24 hours post-treatment. The effects of ascorbic acid analogs on wound closure exhibited various patterns. Magnesium ascorbyl phosphate (MAP) promoted wound closure to a similar extent as ascorbic acid, and its effect was further enhanced by glycinamide. Other analogs, such as 3-O-ethyl ascorbic acid (EAA), ascorbyl 2-O-glucoside (AG), and ascorbyl tetraisopalmitate (ATI), slightly promoted wound closure in the early stage (24 hours) but inhibited it in the later stages (36 and 48 hours). Treatment with glycinamide alone promoted wound closure, and this effect was further enhanced by combined treatment with magnesium ascorbyl phosphate (MAP), but not with 3-O-ethyl ascorbic acid (EAA), ascorbyl 2-O-glucoside (AG), or ascorbyl tetraisopalmitate (ATI). These results indicate that each ascorbic acid analog has different effects on wound closure and that glycinamide promotes wound closure under various conditions in the absence or presence of ascorbic acid analogs or TGF-β1.

### 9. The effect of triterpenoid components of Centella asiatica L. on collagen production in human dermal fibroblasts.

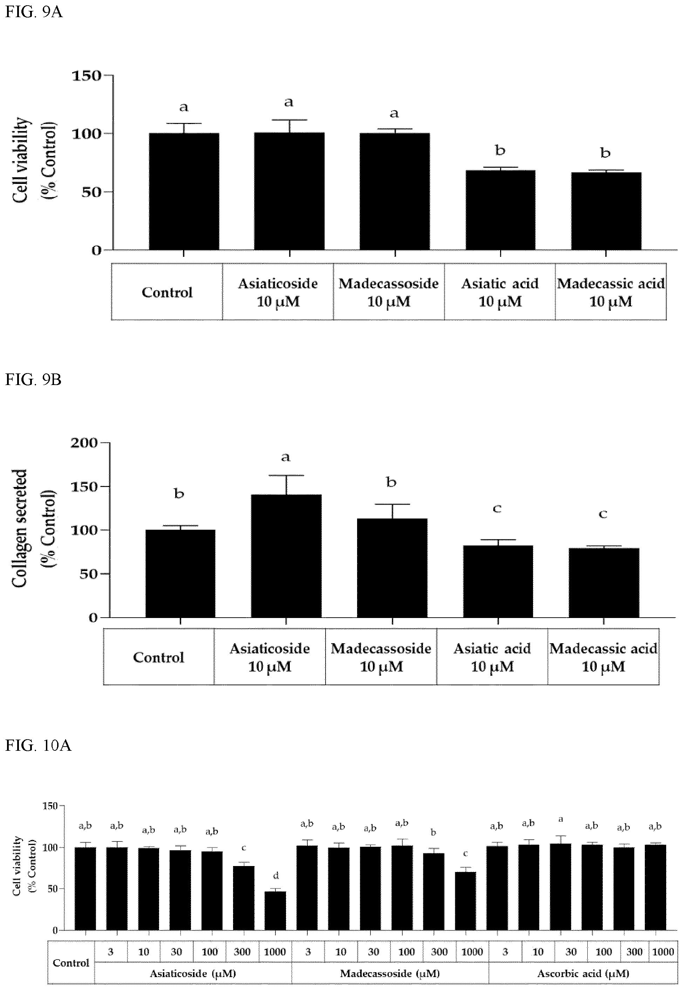

The wound healing promotion effect of Centella asiatica extract is related to collagen production, improvement of angiogenesis, and inhibition of inflammation, with the main pharmacologically active components, triterpenoids such as asiaticoside, madecassoside, asiatic acid, and madecassic acid are reported. In the following experiment, the effects of these four components on cell viability and collagen production were compared (Figures 9a, 9b). When cells were treated with each compound at 10 µM, asiatic acid and madecassic acid both decreased cell viability and collagen production, while madecassoside had no significant effect on either. In contrast, asiaticoside significantly increased collagen production without affecting cell viability.

### 10. Comparison of the effects of Asiaticoside, Madecassoside, and Ascorbic Acid on collagen production in human dermal fibroblasts.

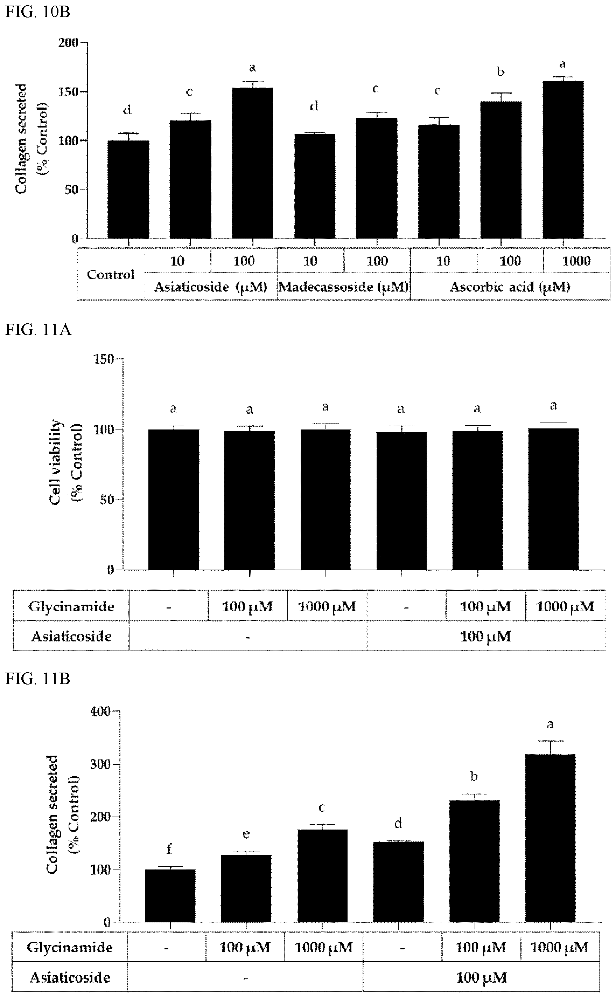

In the following experiment, the effects of asiaticoside, madecassoside, and ascorbic acid on cell viability and collagen production were evaluated at various concentrations (Figures 10a, 10b). Asiatic acid and madecassic acid had no effect on cell viability at concentrations below 100 µM, but reduced cell viability at concentrations above 300 µM, whereas ascorbic acid had no effect on cell viability up to a concentration of 1000 µM. Therefore, the effects of these compounds on collagen production at non-cytotoxic concentrations were compared. Madecassoside increased collagen production at a concentration of 100 µM, asiaticoside increased collagen production at concentrations of 10-100 µM, and ascorbic acid increased collagen production at concentrations of 10-1000 µM. The effect of asiaticoside at 100 µM was statistically comparable to the effect of ascorbic acid at 1000 µM.

### 11. The effect of individual or combined treatment of asiaticoside and glycinamide on collagen production in human dermal fibroblasts.

The cell viability and collagen production were compared when treated individually or in combination with asiaticoside and glycinamide (Fig. 11a, 11b). The change (131.7%) due to the combined treatment of 100 µM asiaticoside and 100 µM glycinamide was significantly greater than the arithmetic sum of the changes from individual treatments of 100 µM asiaticoside and 100 µM glycinamide (51.8% + 27.2% = 79.0%). The change (218.7%) due to the combined treatment of 100 µM asiaticoside and 1000 µM glycinamide was significantly greater than the arithmetic sum of the changes from individual treatments of 100 µM asiaticoside and 1000 µM glycinamide (51.8% + 75.7% = 127.5%). This result shows that the combined treatment of asiaticoside and glycinamide synergistically promotes collagen production in cells.

### 12. The effect of glycine, proline, lysine, or their amidated amino acids on collagen production in various cells.

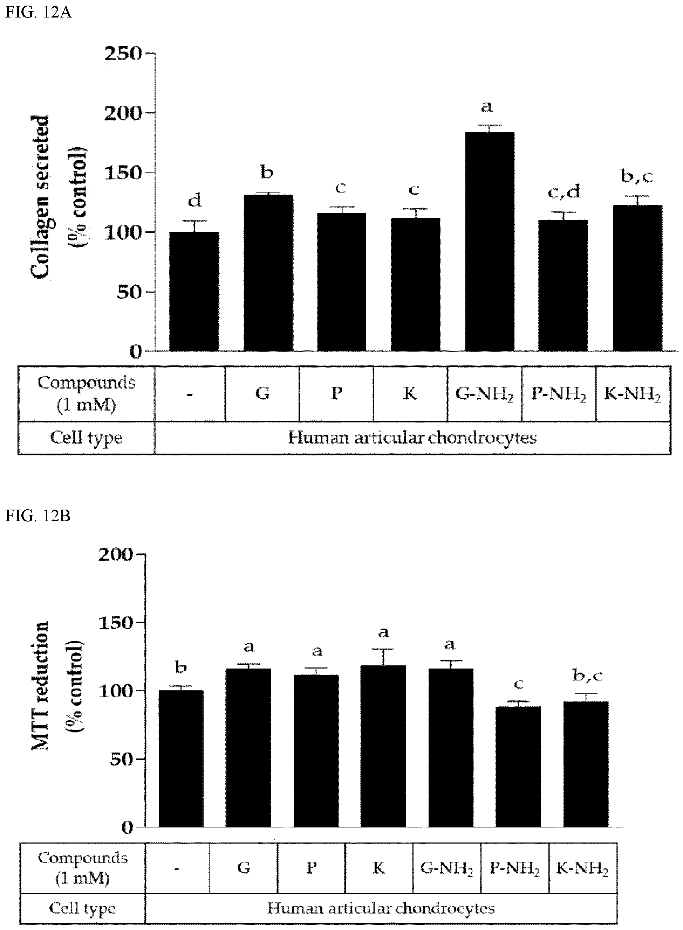

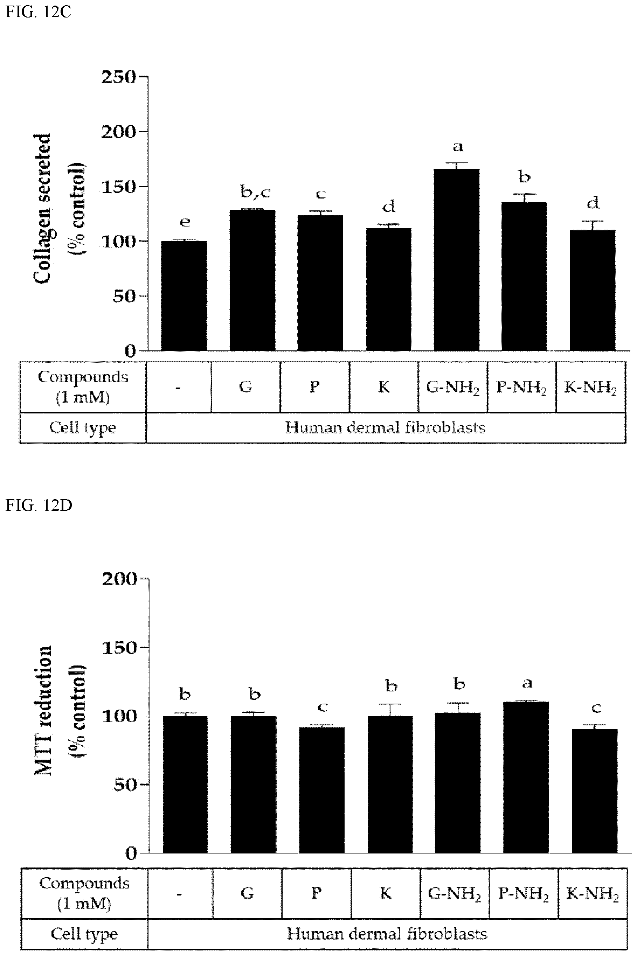

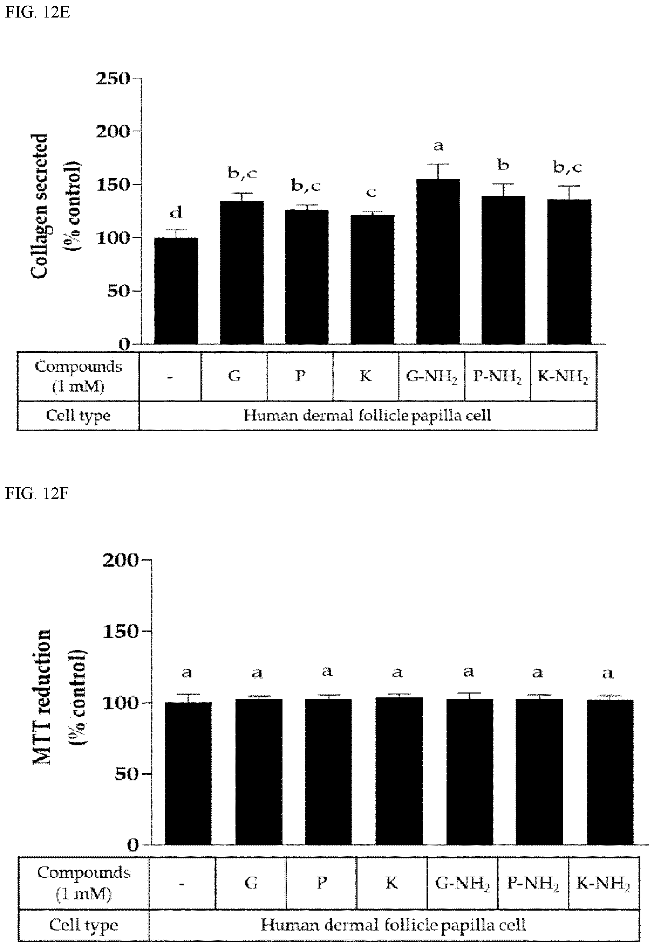

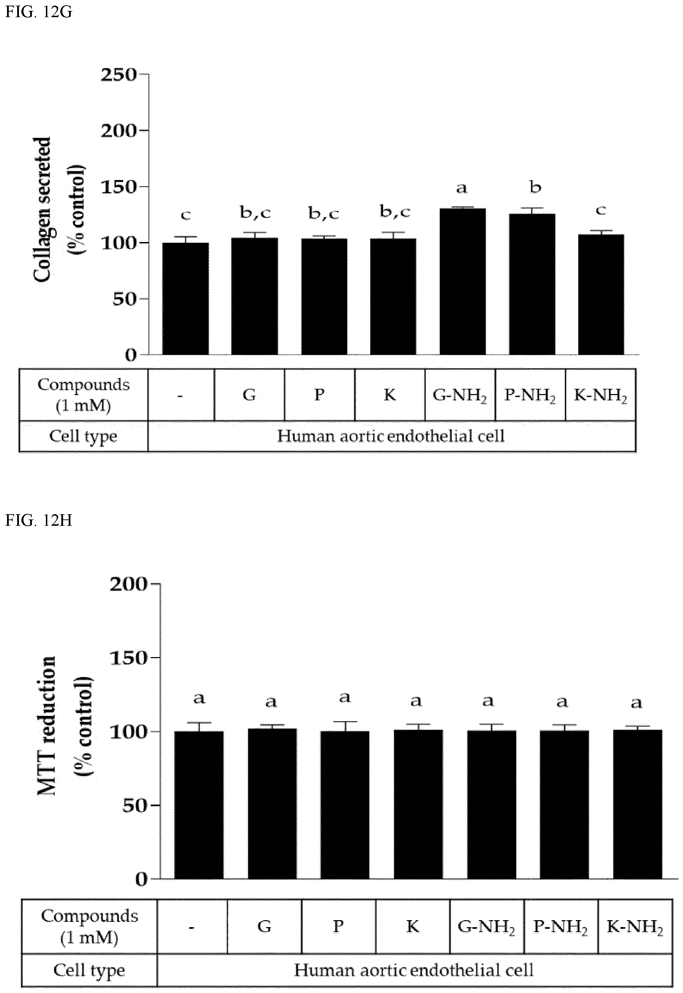

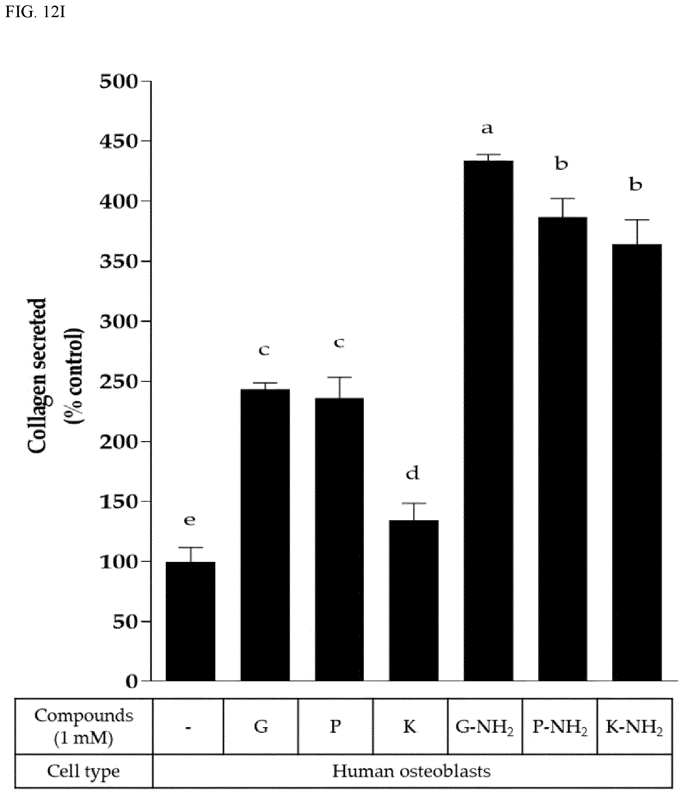

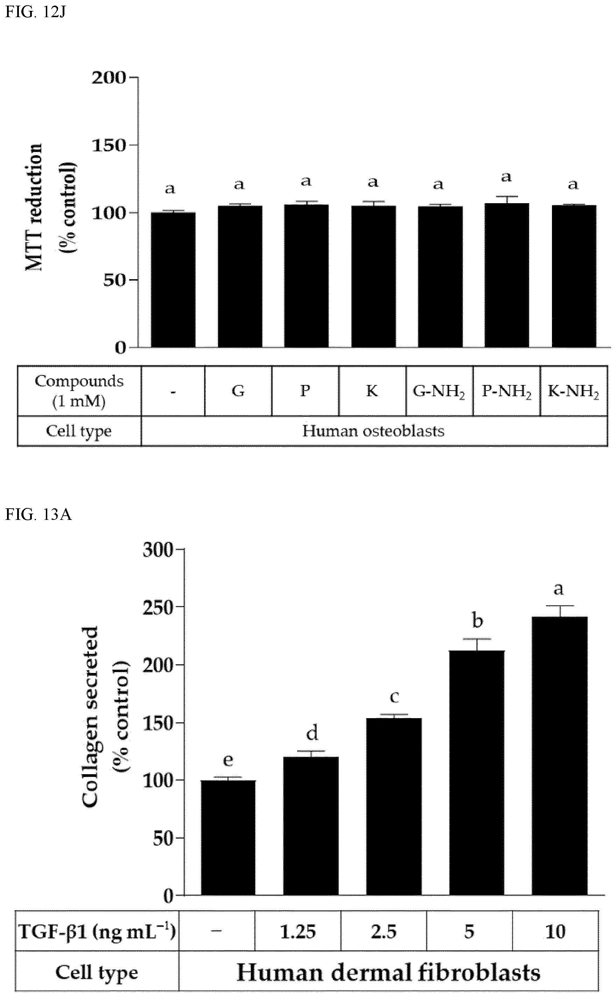

To investigate the effects on collagen production in various cells, human articular chondrocytes (HAC), human follicle dermal papilla cells (HFCPC), human aortic endothelial cells (HAoEC), and human osteoblasts (HOB) were individually treated with glycine (G), proline (P), lysine (K), and their amidated amino acids glycinamide (G-NH₂), prolinamide (P-NH₂), and lysinamide (K-NH₂) at a concentration of 1 mM. As shown in Figures 12a to 12j, although there was no significant difference in cell viability, the amidated amino acids exhibited superior collagen production effects compared to the free amino acids in various cells, with glycinamide being the most effective among the amidated amino acids. Notably, the collagen production effect of glycinamide in HOB was 433.8%. Therefore, glycinamide was confirmed to most effectively enhance collagen production in various cells.

### 13. Concentration-dependent effects of TGF-β1, glycinamide, and glucosamine on human dermal fibroblasts and human articular chondrocytes.

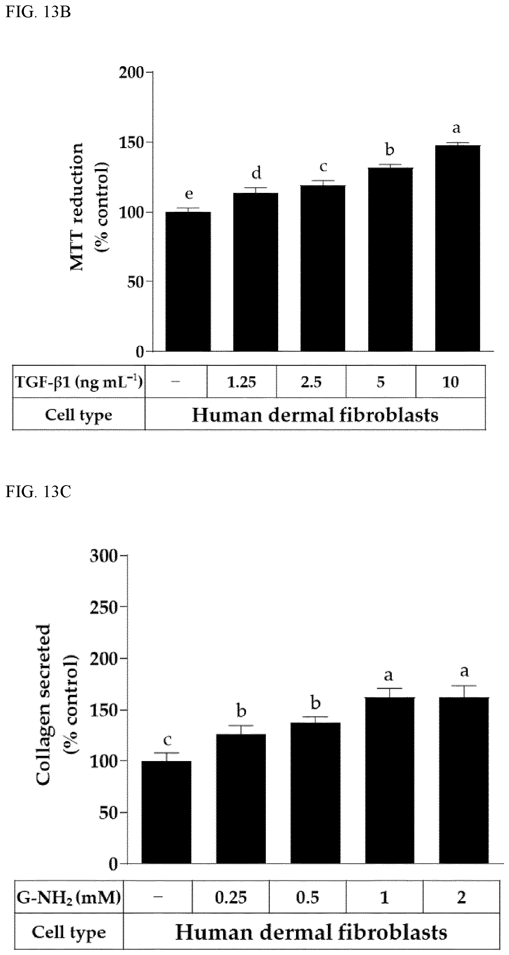

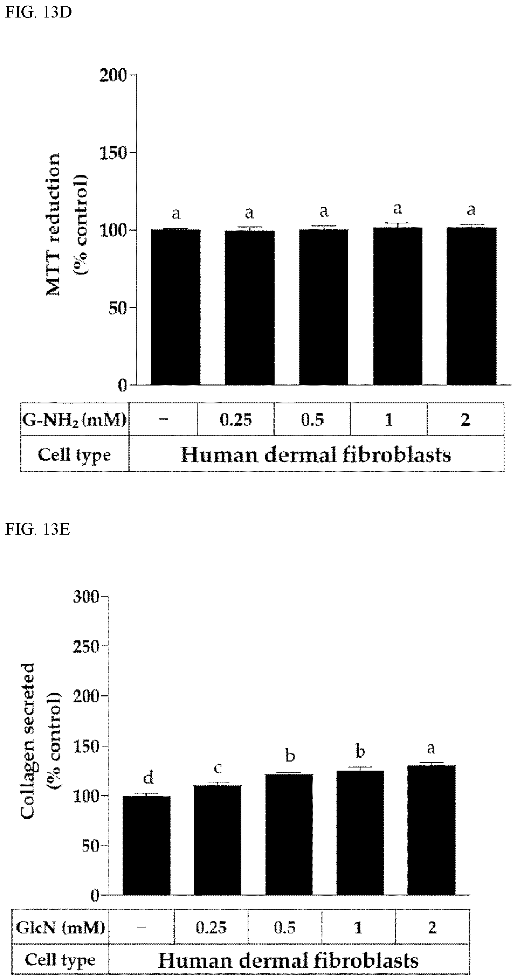

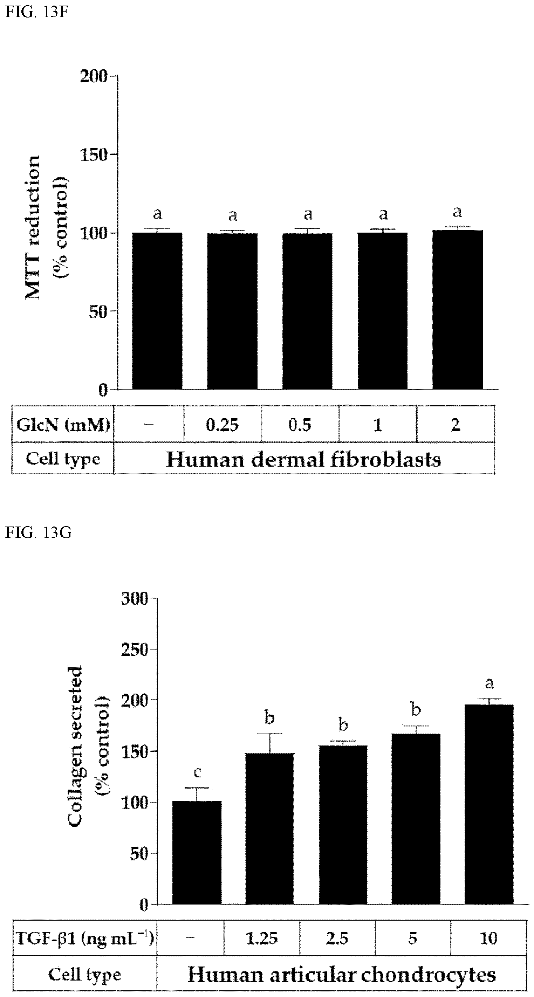

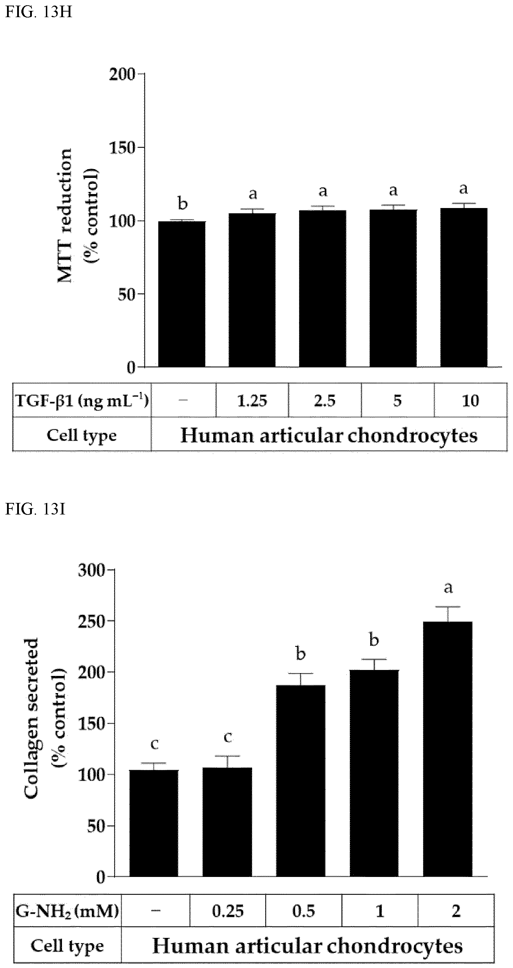

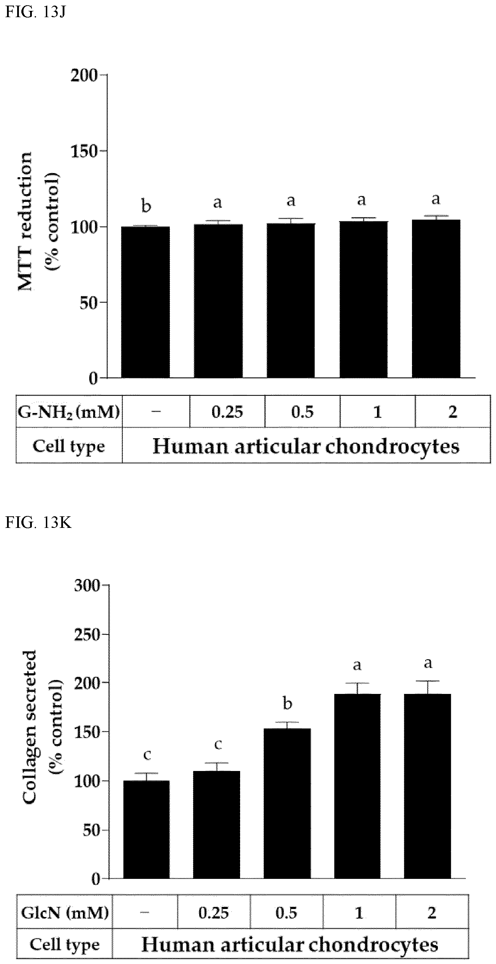

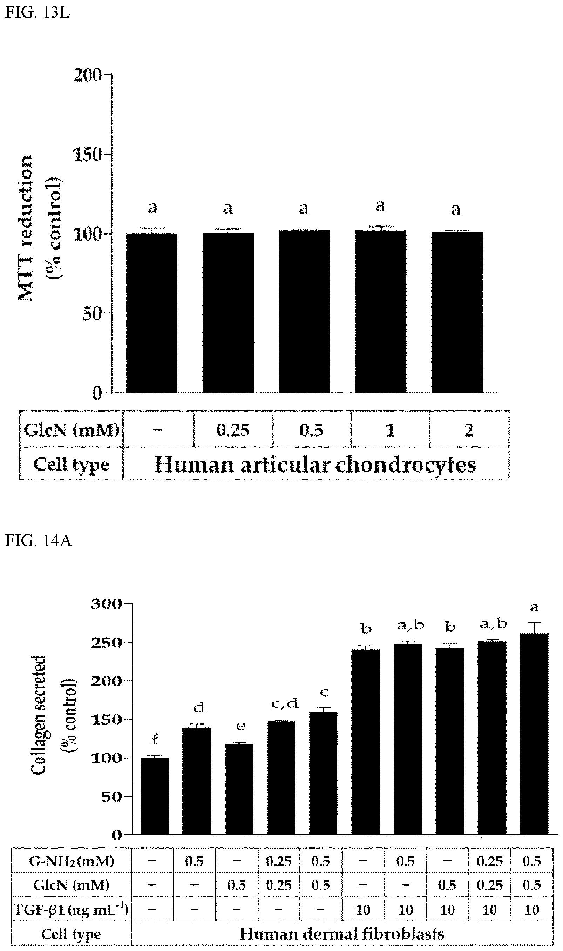

The effects of TGF-β1, glycinamide (G-NH₂), and glucosamine (GlcN) on cell proliferation and collagen production were compared in two types of cells. As shown in Figures 13a to 13f, TGF-β1 increased the proliferation of human dermal fibroblasts in a concentration-dependent manner, whereas glycinamide and glucosamine did not affect cell proliferation. As expected, TGF-β1 exhibited a collagen production enhancement effect that surpassed its cell proliferation effect. Glycinamide increased collagen production in a concentration-dependent manner without affecting cell proliferation. Similarly, glucosamine increased collagen production without affecting cell proliferation.

As shown in Figures 13g to 13l, 0.5 mM glucosamine in human articular chondrocytes increased collagen production without affecting cell viability. Glycinamide and TGF-β1 slightly but significantly increased cell viability compared to the control group, with glycinamide enhancing collagen production at a concentration of 0.5 mM and showing the greatest effect at 2 mM with an increase of 238.6%. TGF-β1 improved collagen production in a dose-dependent manner up to 10 ng mL-1.

### 14. The effect of individual or combined treatment with glycinamide, glucosamine, and TGF-β1 on collagen production in human articular chondrocytes and human dermal fibroblasts.

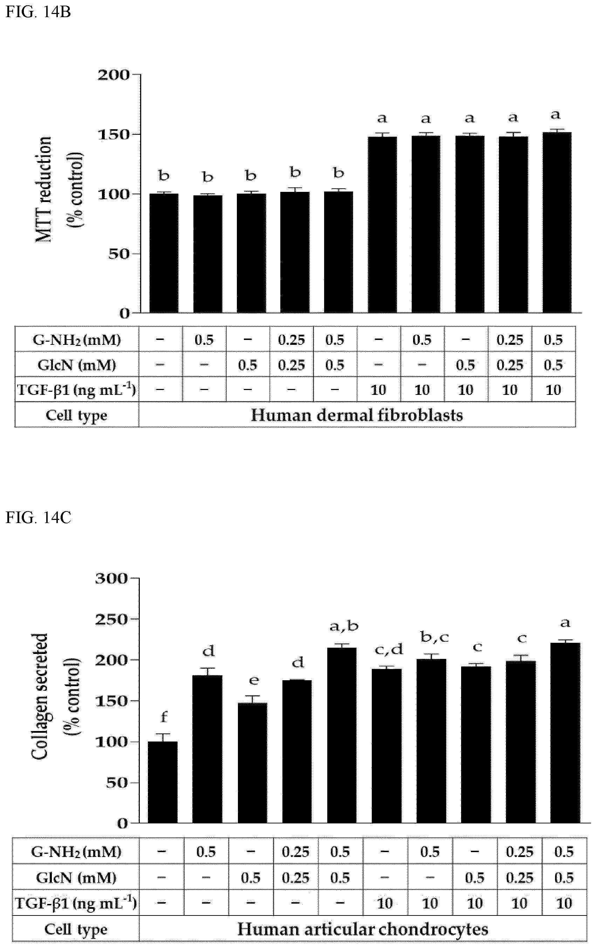

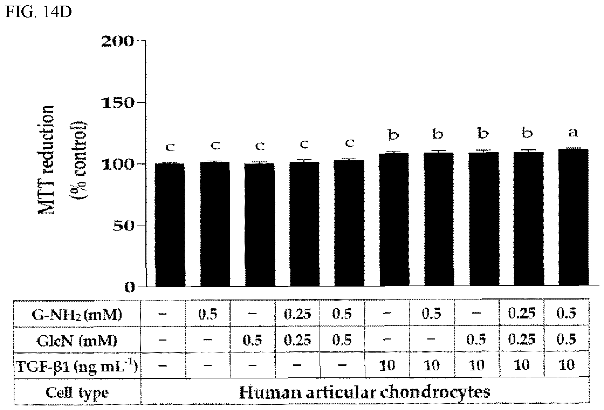

Since glycinamide (G-NH₂) and glucosamine (GlcN) have each been observed to promote collagen production, we further investigated whether their combined treatment exhibits a synergistic effect in the absence and presence of TGF-β1. As shown in Figures 14a to 14d, in the absence of TGF-β1, glycinamide (0.5 mM) and glucosamine (0.5 mM) increased collagen production in human articular chondrocytes by 181.3% and 147.6%, respectively, compared to the control, and their combined treatment increased collagen production in a concentration-dependent manner. In human dermal fibroblasts, glycinamide (0.5 mM) and glucosamine (0.5 mM) increased collagen production by 139.2% and 118.6%, respectively, compared to the control, and their combined treatment increased collagen production in a concentration-dependent manner. These experimental results demonstrate that the combined treatment of glycinamide and glucosamine synergistically promotes collagen production. TGF-β1 (10 ng mL⁻¹) alone showed an effect of enhancing collagen production in human articular chondrocytes and human dermal fibroblasts. The combined treatment of glycinamide (0.5 mM) and glucosamine (0.5 mM) in human articular chondrocytes exhibited a superior effect on collagen production enhancement compared to TGF-β1 (10 ng mL⁻¹). The collagen production enhancement effect of individual or combined treatment of glycinamide and glucosamine was greater in the absence of TGF-β1 than in its presence. The combined treatment of glycinamide and glucosamine and the treatment of TGF-β1 in human dermal fibroblasts and human articular chondrocytes resulted in better cell proliferation compared to the control.

### INDUSTRIAL APPLICABILITY

The composition of the present invention comprising amidated amino acids is highly industrially applicable as it can be very useful in the development of treatments for various diseases and lesions that may arise due to collagen deficiency by promoting collagen production within cells.

## Claims

1. A pharmaceutical composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginemide, prolinamide, glutaminamide, argininamide, threoninamide, valinamide, and tyrosinamide.

2. The pharmaceutical composition according to claim 1, wherein the composition promotes collagen production in cells.

3. The pharmaceutical composition according to claim 2, wherein the cell is selected from the group consisting of fibroblasts, follicular cells, dermal papilla cells, endothelial cells, chondrocytes, muscle cells, and osteoblasts.

4. The pharmaceutical composition according to claim 1, wherein the pharmaceutical composition is the composition forthe prevention or treatment of diseases or lesions selected from the group consisting of skin elasticity reduction and wrinkles due to aging; hypertension and reduced blood flow due to decreased vascular elasticity; decreased ligament elasticity; decreased joint elasticity; elastosis, dermal-epidermal atrophy, dermal atrophic skin diseases due to photoaging; wounds; burns; skin lesions; pressure ulcers; dermal aplasia induced by drug administration; bone and tooth developmental disorders, osteoporosis; periodontal diseases; and hair loss.

5. The pharmaceutical composition according to claim 1, wherein the pharmaceutical composition further comprises at least one selected from the group consisting of ascorbic acid, asiaticoside, glucosamine, and their analogs and derivatives.

6. The pharmaceutical composition according to claim 5, wherein the ascorbic acid analog is selected from the group consisting of ascorbic acid, a salt of ascorbic acid, magnesium ascorbyl phosphate, 3-O-ethyl ascorbic acid, ascorbyl glucoside, and ascorbyl tetraisopalmitate.

7. The pharmaceutical composition according to claim 5, wherein the asiaticoside analog is selected from the group consisting of asiaticoside, a salt of asiaticoside, madecassoside, asiatic acid, and madecassic acid.

8. A cosmetic composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginemide, prolinamide, glutaminamide, argininamide, threoninamide, valinamide, and tyrosinamide.

9. The cosmetic composition according to claim 7, wherein the cosmetic composition is a composition for skin wrinkle improvement, skin moisture enhancement, skin elasticity improvement, or skin aging prevention.

10. A food composition for promoting collagen production, comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginemide, prolinamide, glutaminamide, argininamide, threoninamide, valinamide, and tyrosinamide.

11. The food composition according to claim 9, wherein the food composition is a functional food composition.

12. Use of one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginemide, prolinamide, glutaminamide, argininamide, threoninamide, valinamide, and tyrosinamide for the preparation of a composition for promoting collagen production.

13. The use according to claim 12, wherein the composition is for use in promoting collagen production in cells.

14. A method for promoting collagen production, comprising administering to a subject in need thereof an effective amount of a composition comprising one or more amidated amino acids selected from the group consisting of alaninamide, cysteinamide, glutamic acid-alpha-amide, phenylalaninamide, glycinamide, isoleucinamide, lysinamide, leucinamide, methioninamide, asparaginemide, prolinamide, glutaminamide, argininamide, threoninamide, valinamide, and tyrosinamide.

15. The method according to claim 14, wherein the composition is for use in promoting collagen production in cells.
